# EUROPÄISCHE PATENTSCHRIFT

(11) **EP 0 769 004 B1**
(45) Veröffentlichungstag und Bekanntmachung des Hinweises auf die Patenterteilung: **09.04.2003**
(21) Anmeldenummer: 95924256.1
(22) Anmeldetag: 16.06.1995
(51) Int. Cl.: C07D 201/08

(54) **VERFAHREN ZUR HERSTELLUNG VON CYCLISCHEN LACTAMEN**
PROCESS FOR PRODUCING CYCLIC LACTAMES
PROCEDE DE PREPARATION DE LACTAMES CYCLIQUES

(30) Priorität: 28.06.1994 DE 4422610
(43) Veröffentlichungstag der Anmeldung: 23.04.1997
(73) Patentinhaber: BASF AKTIENGESELLSCHAFT, 67056 Ludwigshafen (DE)
(72) Erfinder: FUCHS, Eberhard, D-67227 Frankenthal (DE); WITZEL, Tom, D-67069 Ludwigshafen (DE)
(86) Internationale Anmeldenummer: EP9502338
(87) Internationale Veröffentlichungsnummer: WO96000722

(56) Entgegenhaltungen:
- EP-A- 0 271 815
- DE-A- 1 944 910
- DE-A- 2 111 216
- DE-A- 2 535 689
- DE-A- 3 403 574
- FR-A- 1 184 282
- FR-A- 2 284 596
- US-A- 3 485 821
- US-A- 4 628 085
- IND. ENG. CHEM. PROCESS DES. DEV., Bd.17, Nr.1, 1978 Seiten 9 - 16 MARES, SHEEHAN 'Kinetics of Caprolactam Formation from 6-Aminocaproic Acid, Ester and Amide' in der Anmeldung erwähnt
- TETRAHEDRON LETT., Bd.21, 1980 Seiten 2443 - 2446 BLADE-FONT, A. 'Facile Synthesis of gamma-,delta- and epsilon-Lactams by Cyclodehydration of Amino Acids on Alumina or Silica Gel' in der Anmeldung erwähnt

## Beschreibung

Die vorliegende Erfindung betrifft ein Verfahren zur Herstellung von Caprolactam durch Umsetzung von Aminocapronsäure oder deren C₁-C₄-Alkylester oder 6-Aminocapronsäureamid mit Wasser.

Gegenstand der US 3,485,821 ist die nichtkatalysierte Umsetzung von Aminocapronsäure, Aminocapronsäureamid und alkylsubstituierte Derivate davon zu Caprolactam oder alkylsubstituiertes Caprolactam, indem man die Umsetzung in Wasser bei einer Temperatur im Bereich von 150 bis 350°C durchführt, wobei man dem Wasser Ammoniumsalze zusetzen kann. Die Umsätze zu Caprolactam liegen jedoch nicht höher als 90%.

Die DE-A 2,535,689 beschreibt ein Verfahren zur Herstellung von Caprolactam durch Umwandlung von in Methanol oder Ethanol gelöster 6-Aminocapronsäure, wobei das Lösungsmittel einen Alkoholgehalt von mindestens 60 Vol.-% aufweist und man die Reaktion bei einer Temperatur im Bereich von 170 bis 200°C durchführt. Höhere Temperaturen, insbesonders bei 220°C und höher, führen nach der DE-A 2,535,689 zur vermehrten Bildung des entsprechenden Alkylesters der 6-Aminocapronsäure, was letztlich zur vermehrten Oligomerbildung führt. Wassergehalte über 40 Vol.-% sollen gemäß diesem Dokument zur Bildung offenkettiger Polyamide führen. Für die gewerbliche Nutzung ist die Zugabe der 6-Aminocapronsäure ein großer Nachteil, da die Aminosäure vollständig gelöst sein soll, bevor sie zu Caprolactam umgesetzt wird, und somit ständig 6-Aminocapronsäure zugegeben und deren Verbrauch kontrolliert werden muß.

In der Ind.Eng.Chem.Process Des.Dev.,17(1) (1978) 9-16 (Mares et al.) wird die Umsetzung von 6-Aminocapronsäure sowohl in Wasser als auch in Ethanol zu Caprolactam ohne Zusatz eines Katalysators beschrieben. Die Ausbeute an Caprolactam in Wasser wird bei einer Reaktionstemperatur von 211°C mit nur 65% angegeben, während in reinem Ethanol Ausbeuten bis 98% erreichbar sein sollen. Jedoch konnten die von Mares et al. beschriebenen Ausbeutewerte von 92% und höher experimentell in eigenen Versuchen nicht nachvollzogen werden. Vielmehr sind diese hohen Ausbeutewerte nur dadurch erklärbar, daß Mares et al. keine 10 gew.-%ige Lösung von 6-Aminocapronsäure einsetzte, sondern durch kontinuierliches Zugeben von 6-Aminocapronsäure in die ethanolische Lösung minimale 6-Aminocapronsäurekonzentrationen zu Caprolactam cyclisiert hat. Da Caprolactam unter den gewählten Reaktionsbedingungen stabil ist, konnten die hohen Ausbeuten (92 und 98%) nur dadurch erhalten werden, daß sich Caprolactam während der Reaktion anreicherte. Diese Vermutung wird gestützt durch die DE 2,535,689 (mit Mares als Miterfinder). In Beispiel 1 dieses US-Patentes findet sich der Hinweis, daß eine Reaktion der 6-Aminocapronsäure verhindert werden soll, bevor die 6-Aminocapronsäure vollständig gelöst ist. Zu diesem Zweck solle man die 6-Aminocapronsäure so langsam zusetzen, daß sich keine feste Säure im Lösungsmittel befindet, sondern die 6-Aminocapronsäure praktisch unmittelbar in dem heißen Lösungsmittel dispergiert und vollständig gelöst oder während des Aufheizens dispergiert wird. Da sich sowohl in kaltem als auch in siedendem Ethanol (78°C) maximal nur 1,3 g/l = 0,13 Gew.-% 6-Aminocapronsäure lösen, ist verständlich, daß 6-Aminocapronsäure in Mares et al. nur sukzessive zugegeben werden konnte, um eine Lösung zu erhalten, die nur rein rechnerisch 10 Gew.-% 6-Aminocapronsäure enthielt. Im übrigen ist die technische Durchführung einer solchen Dosierung bei 200°C und einem Druck von 80 bar mit sehr großen Schwierigkeiten nebst einem großen Zeitaufwand verbunden.

In Mares et al. (Ind.Eng.Chem.Process Des.Dev., 17(1) (1978) 9-16) wird auch die Umsetzung von 6-Aminocapronsäureethylester in Ethanol zu Caprolactam untersucht. Dabei wird festgestellt, daß eine quantitative Cyclisierung nur bei tiefen Temperaturen und bei hoher Verdünnung möglich ist. Insbesondere sind gemäß Mares et al. aus 6-Aminocapronsäureethylester bei 200°C und einer Reaktionszeit von 10 h eine nur 90%ige Caprolactam-Ausbeute erreichbar. Die Ausbeute läßt sich gemäß Mares et al. bei einer Temperatur von 150°C auf maximal 95% steigern, jedoch sind Reaktionszeiten von 10 h und darüber für eine gewerbliche Anwendung indiskutabel.

Ebenfalls bei Mares et al. wird die Umsetzung von 6-Aminocapronsäureethylester in Wasser zu Caprolactam bei einer Temperatur von 200°C und fünf Stunden Reaktionszeit mit einer Ausbeute von maximal 62.5% beschrieben. In der gleichen Veröffentlichung wird auch die Umsetzung von 6-Aminocapronsäureamid zu Caprolactam sowohl in Wasser als auch in Ethanol untersucht, wobei Ausbeuten von maximal 71% bei über 70 min Reaktionszeit erhalten werden können.

In Tetrahedron Lett. 21 (1980) 2443 wird die Cyclisierung von 6-Aminocapronsäure als Suspension in Toluol in Gegenwart von Aluminiumoxid oder Kieselgel unter Auskreisen des Reaktionswassers beschrieben. Zur vollständigen Desorption des Caprolactams muß der Katalysator mit Methylenchlorid/Methanol gewaschen und Polymere mit Diethylether ausgefällt werden. Die Ausbeute an Caprolactam beträgt 82% an Aluminiumoxid und 75% an Kieselgel, wobei die Reaktionszeit allerdings jeweils 20 h beträgt.

In der EP-A 271 815 wird die Cyclisierung von 6-Aminocapronsäureestern zu Caprolactam beschrieben, wobei man die Ester in einem aromatischen Kohlenwasserstoff löst und dann bei 100 bis 320°C unter gleichzeitiger Abtrennung des gebildeten Alkohols cyclisiert.

Nachteilig sind die Abtrennung des Alkohols unter Druck sowie die aufwendig entfernbaren aromatischen Kohlenwasserstoffe, da hochsiedend, und die langen Reaktionszeiten, beispielsweise 14 h bei Durchführung der Reaktion in o-Xylol (140°C).

In der EP-A 376 122 wird die Cyclisierung von 6-Aminocapronsäureestern in Gegenwart eines aromatischen Kohlenwasserstoffs und Wassers zu Caprolactam bei Temperaturen im Bereich von 230 bis 350°C beschrieben.

Aufgabe der vorliegenden Erfindung war es daher, ein Verfahren zur Herstellung von Caprolactam durch Umsetzung von Aminocapronsäure oder deren C₁-C₄-Alkylester oder 6-Aminocapronsäureamid mit Wasser zur Verfügung zu stellen, das die vorstehend beschriebenen Nachteile nicht mit sich bringt.

Demgemäß wurde ein verbessertes Verfahren zur Herstellung von Caprolactam durch Umsetzung von 6-Aminocapronsäure oder deren C₁-C₄-Alkylester oder 6-Aminocapronsäureamid mit Wasser, dadurch gekennzeichnet, dass man die Umsetzung in flüssiger Phase unter Verwendung von Titandioxid als heterogenem Katalysator in einer Mischung aus Wasser und einem C₁-C₄-Alkanol mit einem Wassergehalt von 1 bis 50 Gew.-% bei einer Temperatur im Bereich von 140 bis 320°C und einer Verweilszeit bis zu 30 Minuten durchführt, gefunden.

Als Ausgangsstoffe im erfindungsgemäßen Verfahren werden Aminocarbonsäureverbindungen der allgemeinen Formel I

H₂N ― (CH₂)₅ ― COR¹ I

in der R¹ für OH, -O-C₁-C₄-Alkyl wie -O-Methyl, -O-Ethyl, -O-n-Propyl, -O-i-Propyl, -O-n-Butyl, -O-sek.Butyl, -O-tert.Butyl, -Oi-Butyl, oder -NH₂ steht, eingesetzt.

Ganz besonders bevorzugt sind 6-Aminocapronsäure, 6-Aminocapronsäuremethylester, 6-Aminocapronsäureethylester und 6-Aminocapronsäureamid.

Die Ausgangsverbindungen sind im Handel erhältlich oder beispielsweise gemäß EP 234,295 und Ind. Eng. Chem. Process Des. Dev. 17 (1978) 9-16 herstellbar.

Nach dem erfindungsgemäßen Verfahren werden die vorstehend beschriebenen Aminocarbonsäureverbindungen mit Wasser in flüssiger Phase unter Verwendung heterogener Katalysatoren zu cyclischen Lactamen umgesetzt. Bei Verwendung von Aminocarbonsäureverbindungen der allgemeinen Formel I erhält man Caprolactam.

Die Umsetzung wird erfindungsgemäß in flüssiger Phase bei Temperaturen im Bereich von 140 bis 320, vorzugsweise von 160 bis 300°C vorgenommen; den Druck wählt man üblicherweise im Bereich von 0,5 bis 25 MPa, jedoch so, daß das Reaktionsgemisch unter den angewandten Bedingungen flüssig ist. Die Verweilzeiten betragen erfindungsgemäß bis zu 30 Minuten.

Erfindungsgemäß setzt man die Aminocarbonsäureverbindung in Form einer 1 bis 50, bevorzugt 5 bis 50 gew.-%igen, besonders bevorzugt 5 bis 25 gew.-%igen Lösung in Wasser/Lösungsmittelgemischen ein. Als Lösungsmittel kommen erfindungsgemäß C₁-C₄-Alkanole wie Methanol, Ethanol, n-, i-Propanol, n-, i-, sek.- und tert.-Butanol in Betracht. Auch Ammoniak kann bei der Reaktion anwesend sein. Selbstverständlich können auch Mischungen organischer Lösungsmittel Anwendung finden.

Als heterogenen Katalysator setzt man erfindungsgemäß Titandioxid als Anatas oder Rutil ein.

Mischungen dieser Oxide untereinander sind ebenfalls möglich.

Die vorstehend genannten Oxide können mit Verbindungen der I. und VII. Hauptgruppe des Periodensystems dotiert sein bzw. diese enthalten.

Die Menge an Katalysator richtet sich in der Regel nach der Verfahrensweise: bei einer diskontinuierlichen Durchführung wählt man die Menge an Katalysator im Bereich von 0,1 bis 50, vorzugsweise von 1 bis 30 Gew.-%, bezogen auf Aminocarbonsäureverbindung der allgemeinen Formel I, bei kontinuierlicher Durchführung wählt man die Katalysatorbelastung im Bereich von 0,1 bis 5 kg/l/h, bezogen auf die Aminocarbonsäureverbindung der allgemeinen Formel I.

Nach dem erfindungsgemäßen Verfahren erhält man cyclische Lactame, insbesondere Caprolactam in hoher Ausbeute.

### Beispiele

### Vergleichsbeispiel 1

In einen geheizten Rohrreaktor von 100 ml Inhalt (Durchmesser 9 mm; Länge 400 mm), der mit Titandioxid (Anatas) in Form von 1,5 mm Strängen gefüllt war, wurde bei 100 bar eine Lösung von 6-Aminocapronsäure in Wasser geleitet. Der den Reaktor verlassende Produktstrom wurde gaschromatographisch und hochdruckflüssigchromatographisch (HPLC) analysiert.

| ACS [%] | Wasser [%] | Temp. [°C] | VWZ [min] | Aus. [%] |
|---|---|---|---|---|
| 10 | 90 | 220 | 15 | 66 |
| 10 | 90 | 220 | 30 | 76 |

### Beispiel 1

Entsprechend den in Beispiel 1 beschriebenen Versuchen wurde eine Lösung von Aminocapronsäure in Ethanol/Wasser durch einen mit Titanoxidsträngen gefüllten Rohrreaktor gepumpt.

| ACS [%] | Wasser [%] | Ethanol [°C] | Temp. [°C] | VWZ [min] | Aus. [%] |
|---|---|---|---|---|---|
| 10 | 40 | 50 | 220 | 15 | 98 |
| 10 | 40 | 50 | 220 | 30 | 90 |

### Beispiel 2

Entsprechend den in Beispiel 1 beschriebenen Versuchen wurde eine Lösung von Aminocapronsäureethylester in Ethanol in Gegenwart von Wasser durch einen mit Titanoxidsträngen gefüllten Rohrreaktor gepumpt.

| ACSE [%] | Wasser [%] | Ethanol [°C] | Temp. [°C] | VWZ [min] | Ums. [%] | Sel. [%] | Ausbeute [%] |
|---|---|---|---|---|---|---|---|
| 10 | 1,1 | 88,9 | 220 | 15 | 97 | 93 | 90 |
| 10 | 1,1 | 88,9 | 220 | 30 | 97 | 85 | 83 |
| 10 | 1,1 | 88,9 | 220 | 60 | 100 | 79 | 79 |
| 10 | 4,4 | 85,6 | 220 | 30 | 97 | 95 | 92 |

### Vergleichsbeispiel 2

Gemäß Ind. Eng. Chem. Process Des. Dev. 17 (1978) Seite 16 wurden jeweils eine 10 gew.-%ige Lösung von 6-Aminocapronsäure in Ethanol bei unterschiedlichen Verweilzeiten auf 200°C erhitzt. In der untenstehenden Tabelle sind die Ergebnisse aufgelistet.

| VWZ [min] | Temp. [°C] | Umsatz [%] | Selektivität [%] | Ausbeute an Caprolactam[%] |
|---|---|---|---|---|
| 10 | 200 | 90 | 80 | 72 |
| 15 | 200 | 87 | 93 | 80 |
| 20 | 200 | 90 | 90 | 81 |
| 30 | 200 | 90 | 91 | 82 |
| 40 | 200 | 91 | 88 | 80 |

Ausbeuten über 82 % wurden nicht erreicht.

## Patentansprüche

1. Verfahren zur Herstellung von Caprolactam durch Umsetzung von 6-Aminocapronsäure oder deren C₁-C₄-Alkylester oder 6-Aminocapronsäureamid mit Wasser, **dadurch gekennzeichnet, dass** man die Umsetzung in flüssiger Phase unter Verwendung von Titandioxid als heterogenem Katalysator in einer Mischung aus Wasser und einem C₁-C₄-Alkanol mit einem Wassergehalt von 1 bis 50 Gew.-% bei einer Temperatur im Bereich von 140 bis 320°C und einer Verweilzeit bis zu 30 Minuten durchführt.

## Claims

1. A process for the preparation of caprolactam by reacting 6-aminocaproic acid, or the C₁-C₄-alkyl ester thereof, or 6-aminocaproamide with water, **characterized in that** the reaction is carried out in the liquid phase using titanium dioxide as heterogeneous catalyst in a mixture of water and a C₁-C₄-alkanol having a water content of from 1 to 50% by weight at from 140 to 320°C during a residence time of up to 30 minutes.

## Revendications

1. Procédé de préparation de caprolactame par réaction d'acide 6-aminocaproïque ou de ses alkyl(C₁ - C₄)esters ou d'amide 6-aminocaproïque avec de l'eau, **caractérisé en ce que** l'on entreprend la réaction en phase liquide en utilisant du dioxyde de titane comme catalyseur hétérogène, dans un mélange d'eau et d'un alcanol en C₁ à C₄ avec une teneur en eau de 1 à 50% en poids, à une température de l'ordre de 140 à 320°C et avec un temps de séjour jusqu'à 30 minutes.
